# EUROPEAN PATENT APPLICATION

(11) **EP 3 001 173 A1**
(43) Date of publication of application: **30.03.2016**
(21) Application number: 15163145.4
(22) Date of filing: 25.05.2012
(51) Int. Cl.: G01N 11/04, G01N 33/49, B01L 3/00

(54) **CAPILLARY FLUID FLOW MEASURMENT AND CAPILLARY FLOW DEVICE THEREFORE**

(30) Priority: 01.06.2011 GB 201109203; 23.08.2011 GB 201114585
(62) Divisional of application: 12725143.7
(71) Applicant: Carclo Technical Plastics Limited, Ossett, Yorkshire WF5 9WS (GB)
(72) Inventor: Williamson, Ian, Rutland, LE15 6JE (GB); Allen, Gerald, John, Caythorpe, Lincolnshire NG32 3DF (GB)
(74) Representative: HGF Limited

(57) **Abstract**

The present invention relates to a liquid flow device, in particular a capillary testing device provided as a chip, comprising a second pathway which intersects the first pathway at a downstream point of convergence, so that the two pathways share an outlet and when liquid in the second pathway reaches the point of convergence, liquid flow in the first pathway stops. Means for measuring the distance travelled by liquid in the first pathway are provided to determine the extent of liquid flow and to enable correlation with the amount of analyte in the liquid.

## Description

### Field of the invention

This invention relates to the control of liquid flow, with particular, but not exclusive, application in controlling the flow of liquid in a microfluidic device. In particular, a device and method are provided for the quantitative measurement of a substance of interest, for example an analyte.

### Background to the invention

Microfluidic systems are constructs which facilitate a series of operations and manipulations involving liquids to be performed within an integrated device. They are becoming of increasing importance in assays for the detection or measurement of substances of interest (e.g analytes) in samples, where their features (including miniaturisation, reduced reagent usage and ability to perform all the steps of an assay in a small, self-contained device) are advantageous. In particular, microcapillary devices (with channels typically less than 1mm) are gaining acceptance in the field of *in vitro* diagnostics, tests for the measurement of analytes in biological fluids as an aid in the diagnosis and monitoring of healthcare conditions in humans and animals. Such devices are often known as "Lab on a Chip".

A wide variety of assays have been applied to microfluidic systems, including those involving agglutination, coagulation and measurement of particles. In many of these systems, sample reacts with a reagent or reagents within the channel(s), causing a change in the physical properties of the fluid leading to a reduction or cessation of flow along the channel which can be used to determine the presence and/or concentration of the analyte in the sample.

US 3,951,606 (Geomet) describes a quantitative format using this approach for determination of blood coagulation/clotting time. Sample was mixed with appropriate reagent and caused to flow down a tube by gravity. The blood was caused to coagulate by the reagent, and the time to coagulate was determined by how far the fluid front reached before the coagulum caused flow to stop. Gradations on the tube assisted in measuring the distance flowed. A similar approach, based around the use of a capillary, is described by US2002/0187071 (Portascience Inc). Similarly, WO96/00390 describes a microfluidic device for determining blood clotting, where graduations are provided along the channels as markers.

Various patent specifications describe measurement of the reduction in fluid flow (not merely the cessation) as a measure of the degree of agglutination, and thus a mechanism to obtain quantitative measurements. A variety of methods have been described to determine the flow rate, including time to reach a set point or points. EP 483117 (Biotrack) describes such a system.

For any microfluidics-based assay, control of timing, fluid flow, detection/measurement of fluid within the capillaries, etc is desirable. In many assay formats, control of and/or compensation for variations in sample properties, temperature, etc is also desirable.

Many mechanisms have been developed to address these requirements, but unfortunately many of these mechanisms are complex (often requiring some form of instrumentation or reader) which partly negates the advantages of simplicity afforded by the microfluidic device. The majority of approaches developed to date are concerned with direct interaction with the fluid front, which frequently adds complexity to the system. A variety of methods have been employed to control the flow of fluid through capillaries, including modulation of external propulsive force (e.g, external pumps to move fluid through the channels, e.g. US 4,244,694), mechanical control of flow (e.g. EP01194696 (Gyros) describes a system utilising a plug or plugs of intelligent polymer within the capillary to control flow), adjustment of capillary surface tension at a change of dimension (restriction, expansion, shape, etc) (see for example US 2004/0231736, US 5,286,454 and US 5,472,671 (Nilsson) and US 6,143,248 (Tecan)), and modulation of surface property (hydrophobic zones, dissolvable barriers, etc, as described for example in GB 2341924, and US 7,615,191; 7,445,941; 6,271,040; 6,143,576; 6,019,944; 5,885,527; 5,458,852 (Biosite)). Often, fluid control involves a combination of these. All of the approaches summarised in the above prior art rely on the fluid directly contacting the control means.

Thus, there are numerous ways to control fluid flow within a microfluidic device. Those reliant on external control require additional complexity (and thus cost), whilst the passive systems rely on precise shape or physical treatment for control, making manufacture more difficult and less robust.

Many agglutination assays rely upon measuring cessation of flow. However, in certain circumstances, although the clotting or agglutination reaction may cause a blockage of flow, the fluid may continue to "creep" slowly along the channel due to the strong capillary forces. Thus, for example, a "positive" reaction may become "negative" if the device is left for a long period. WO2008/025945 (Alere) describes a system based on the use of two or more capillaries which converge at a junction, and one or more indicator regions which are activated in the presence of liquid. One capillary (test lane) contains reagent(s) which alter the flow of liquid in the presence of analyte, the other (control lane) does not contain analyte-specific reagents. When fluid in one or other of the channels reaches the junction, it prevents further flow of fluid in the other channel because it blocks the egress of air from the channel. Thus, for example, in the presence of analyte, fluid in the test lane will be retarded and so will not reach an indicator region located distally in the channel and so no signal is produced; in the absence of analyte fluid in the test channel will reach the indicator zone and a signal is produced. A variety of formats and locations of indicator regions are described, but all are binary (i.e. analyte is present or not).

There exists a need for a simple, intrinsic mechanism to control fluid flow, whilst providing the basis for quantitative measurement of fluid flow.

### Summary of the invention

Thus, in a first aspect of the invention there is provided a liquid flow device comprising a first pathway having an inlet, and a second pathway having an inlet, the first and second pathways intersecting at a downstream point of convergence and having a common outlet, and means for measuring the extent of liquid flow along the first pathway.

In a further aspect of the present invention, there is provided a capillary testing device, comprising a first capillary pathway having an inlet and a second capillary pathway having an inlet, the first and second capillary pathways intersecting at a downstream point of convergence and having a common outlet, and means for measuring the extent of liquid flow along the first capillary pathway.

In another aspect there is provided a method of measuring the extent of liquid flow along a first pathway is intersected by a second pathway at a downstream point of convergence, such that the first and second pathways having a common outlet, comprising causing or permitting liquid to flow in the first pathway and causing or permitting liquid to flow in the second pathway at least until it reaches the point of convergence, and measuring the extent to liquid flow in the first pathway once the liquid in the second pathway has reached the point of convergence.

In another aspect, the invention provides a method of measuring the extent of liquid flow along a first pathway of a liquid flow device, where the first pathway extends from a sample application region to an outlet and is intersected at a downstream point of convergence by a second pathway which extends from a fluid application region, such that the first and second pathways share a common outlet, wherein the method comprises causing or permitting liquid to flow in the first pathway and causing or permitting liquid to flow in the second pathway at least until it reaches the point of convergence, and measuring the extent to liquid flow in the first pathway once the liquid in the second pathway has reached the point of convergence.

Thus, in another aspect the present invention provides a method which comprises:
i) providing a liquid flow device comprising a first pathway having an inlet and a second pathway having an inlet, the first and second pathways intersecting at a downstream point of convergence and having a common outlet, and means for measuring the extent of liquid flow in a first pathway;
ii) causing or permitting liquid to flow in the first and second pathways from the respective inlet toward the outlet;
iii) using the measuring means to determine the extent of liquid flow along the first pathway, preferably once liquid in the second pathway has reached the point of convergence.

Preferably, the methods of the invention further comprise the step of correlating the extent of liquid flow in the first pathway to the amount of substance of interest in the liquid (sample) of the first pathway, for example using a chart correlating distance and amount for known samples.

In another aspect of the invention, there is provided a kit for measuring the amount of substance of interest in a sample, the kit comprising a liquid flow device as defined herein, a chart correlating distance travelled and concentration of substance of interest, and optionally buffers and reagents.

When liquid is flowing in the second pathway, provided the liquid is upstream of the point of convergence, liquid can flow in the first pathway. However, when liquid in the second pathway reaches the point of convergence, this blocks the outlet from the first pathway and prevents further flow of liquid in the first pathway, as air can no longer escape from the first pathway via the common outlet. Liquid can continue to flow in the second pathway. By arranging the liquid flow rates so that liquid in the second pathway reaches the point of convergence before liquid in the first pathway reaches this point (as determined by factors including the geometry and architecture of the two capillary pathway and the viscosity of the liquids in the passages), the flow of liquid in the second pathway can be used to control the flow of liquid in the first pathway. The liquid in the second pathway thus acts indirectly on the liquid flow in the first pathway.

Thus, for example, the liquid flow in the first pathway can be stopped at the point when the liquid in the second pathway reaches the point of convergence. This point is an appropriate time at which the extent of flow of liquid in the first pathway from the inlet toward the outlet can be measured. This is a measurement of the distance travelled from the inlet. This has the advantage that liquid flow in the first pathway, typically a test pathway, is stopped and does not "creep", such that less false results are obtained. Thus, a method typically involves determining the extent of liquid flow in the first pathway after the liquid in the second pathway has passed the point of convergence and flow in the first pathway has stopped. Preferably, a method of the invention may further comprise using the measurement of distance travelled to determine the amount of a substance of interest in a sample.

Thus, herein reference to the extent of flow in a pathway means the distance travelled by the liquid along the pathway from an inlet toward an outlet.

The invention thus provides a very simple means of accurately measuring the amount of substance of interest in a sample.

The pathways constitute an enclosed system, to enable liquid flow as desired.

The measurement means (or mechanism for measuring) may comprise distance markings provided in relation to at least the first pathway, to measure the distance liquid has travelled, preferably once the liquid in the second pathway reaches the point of convergence.

Any suitable measurement means or mechanism may be provided in the device to measure the extent the liquid in a first pathway has travelled and may represent distance or analyte concentration (determined by means of a predetermined dose response). Where the measurement mechanism comprises distance markings may be provided in any suitable unit (mm, cm, inches or fractions of inches for example), on a linear, logarithmic or other scale. Alternatively, a machine vision system may be used. Use of distance markings for visual reading provides a simple, cheap approach.

In a simple case, distance markings may be provided along at least part of the length of the first pathway. The extent of flow can be determined by reference to the markings, either by eye or by a machine vision system. Preferably, distance markings may extend along at least the part of the first pathway until the point of convergence with the second pathway. Preferably, distance markings are provided from the inlet of a pathway to a point of convergence with a second pathway. In some embodiments, measuring means may be provided in relation to a third pathway, for example for the purpose of making relative measurements.

In an embodiment, the measuring means may comprise a cursor, for example for measuring relative liquid flow. A cursor is preferably an elongate member, having provided thereon distance markings. Preferably, the markings are provided along at least one elongate edge thereof, to be positioned adjacent to a pathway. Preferably, the cursor is movable (preferably slidable) with respect to a pathway, for example slidable within a groove provided in the device. A cursor may be located along a linear portion of a pathway, more preferably between linear portions of two or more pathways (e.g. two first pathways or a first pathway and a third pathway). In any device, two or more cursors may be provided, as appropriate.

Alternatively, the cursor may be fixed relative to a pathway, with a movable pointer (or other indicator) which is movable (e.g slidable) with respect to the cursor.

For convenience, a cursor is provided with a pointer, which can be used to align the fluid front with the cursor, to obtain an accurate measurement of distance travelled. As appropriate, in any cursor two or more pointers may be provided. Preferably, a pointer is movable with respect to the cursor, preferably slidable with respect thereto. A pointer or other indicator may be provided on the opposite edge of the cursor to the distance markings, or on the same edge, preferably at or near one end thereof.

Where a groove is provided, in any one groove, two or more cursors may be provided. For any pathway, two or more cursors may be provided.

It is also envisaged that distance markings may also be provided on the device, which align with those provided on the cursor, so that a measurement of the total distance travelled can be taken.

The cursor should be sufficiently long to accommodate expected flow differential in use of any particular device. After liquid flow in the first pathway has stopped, the cursor is positioned with the indicator (pointer) aligned with the leading edge of liquid flow. At this point, a measurement of distance travelled from the inlet can be taken. Where relative distance is being measured, the position of liquid in another (e.g control) pathway can then be determined by reference to the measurement markings, for example using a second indicator or pointer to mark the leading edge of liquid flow. A moveable cursor enables analyte concentration to be determined very simply and accurately with little risk of error.

The method of the invention can thus be performed using the device of the invention.

The invention can be used to perform a variety of functions, including (but not limited to) regulation of fluid flow (start/stop), control of timing (e.g. of an assay), compensation mechanisms for normalising for sample variations e.g. variations in viscosity, variations due to temperature etc., and providing the basis for measurement of liquid flow.

The invention thus has particular application in microfluidic devices and systems, e.g. as described above, for instance for performing diagnostic assays on liquid samples, commonly a body fluid such as blood (whole blood or plasma), urine, saliva, cerebrospinal fluid, etc., environmental samples etc., e.g. to detect bacteria in water samples. A further application of the invention is measurement of viscosity of any liquid, e.g. oil, etc.

The present invention also has applicability to liquid flow devices generally, although is preferably a capillary flow device, and finds application as a testing device as defined above. Typically a capillary pathway has a diameter of about 1 mm or less. With capillary pathways, no power source is required to permit or enable liquid flow, thus enabling reduction of complexity and cost. Wider pathways may be used, typically in conjunction with a pump or vacuum source to cause liquid flow, or using gravity flow.

In a preferred embodiment, a device of the invention may comprise a third pathway which functions as a control. The third pathway may share a common inlet with a first pathway, or may have a separate inlet from the first pathway. Preferably, a third (control) pathway is in fluid communication with the sample application region which feeds a first pathway. A third pathway may have a separate outlet to the shared outlet of the first and second pathways, or may share the same outlet. Thus, a third pathway may intersect the first pathway at a downstream point of convergence which is the same or different to the point of convergence between the first and second pathways. If different, the third pathway preferably intersects with the first pathway upstream of the point of convergence between the first and second pathways, such that the fluid in both the first and third pathways can be stopped simultaneously when liquid in the second pathway reaches the point of convergence. A third pathway, or control pathway, may be provided to normalise variability between samples, for example in terms of viscosity. Preferably, a third pathway may comprise measurement means for determining the extent of liquid flow in the third pathway. The measurement means may be shared with a first pathway, e.g. provided between parallel sections the two pathways, or may be provided individually in respect of each pathway. Alternatively, two or more third pathways may share a measurement means. The measurement means are preferably as described herein, and preferably extend along at least part of a third pathway, preferably from the inlet to a point corresponding to a point of convergence on a first pathway.

Typically therefore, a first pathway is a testing pathway; the second pathway is a fluid control pathway (e.g. a timing pathway) and the third pathway a reference pathway. Preferably metering means will be provided in the sample testing device to control sample volume for each capillary passage, for example a side passage associated with each capillary passage, as defined herein.

A device of the invention may comprise a single first pathway or may comprise two, three, four, five or more first pathways. Where two or more first pathways are provided, each may be provided with a set of distance markings, or two or more first pathways may share a set of distance markings. The provision of two or more first pathways allows for simultaneous testing for multiple substances of interest.

In an embodiment of the invention where two or more first pathways are provided, one or more second pathways may be provided. Thus, two or more first pathways may intersect at a point of convergence with a single second pathway, which therefore serves to control flow of liquid in two or more first pathways simultaneously. Thus, two or more first pathways and a second pathway may share a common outlet. Alternatively, each first pathway may intersect at a point of convergence with a separate second pathway. Each pair of first and second pathways in the device will share a common outlet.

The first and second pathways may have a common inlet, e.g. constituted by a well for receiving sample to be tested and/or test reagents.

A liquid test sample is typically supplied to the first pathway, e.g. in known amount, possibly via a sample application region, to flow along the first pathway from the inlet toward the outlet. A known amount of test sample may be supplied to the device, to flow to the first pathway. As a further possibility, the device may include a metering device arranged to supply a known amount of sample to the first pathway; in this case it is not necessary to supply a known amount of sample to the device, but sample should be supplied in at least slight excess of the required amount. The same or a different liquid may also be supplied to the second pathway. Where different, the liquid applied to the second pathway may be a chase buffer or any other liquid suitable for flow in the pathway. The choice of liquid will depend upon the purpose of the device and method, and the proposed use of the second pathway. If a chase buffer, it may be supplied to the second pathway, and possibly also to the first pathway, after the test sample, to reduce the quantity of test liquid required. Thus, the liquids in the first and second pathways may be the same or different. Reagents may be provided in one or more of the pathways, preferably first pathways. The reagent may comprise a test reagent that reacts with a test sample to cause a change in viscosity in a manner which is dependent on the presence and/or quantity of an analyte in the sample. For example, such a test reagent may be provided in the first pathway, e.g. a diagnostic reagent such as an agglutination reagent for use in an agglutination assay, for reaction with the sample liquid in known manner.

A control reagent may be provided in a third pathway, with the control reagent having similar characteristics to the test reagent but not reacting with analyte in the test sample; the control reagent may, however, react with other constituents of the test sample, e.g. substances which can interfere with an assay, which would have the advantageous effect of providing a means for controlling the effect of such interferents.

Reagent may be deposited within a pathway or provided in a chamber in a pathway. Reagent may be deposited in a reconstitutable form or in immobilised form. Any suitable methods may be used for deposition of reagent in a capillary channel. Reagents laid down in a capillary pathway may include, for example, agglutination reagents, antibodies, and labels. Other reagents include buffers, and any other assay components. Any suitable buffer may be used, for example, a solution of Ficoll polymer, preferably a 1 % by weight solution of Ficoll polymer in deionised or distilled water (Ficoll is a Trade Mark), which enables the reaction to be carried out with a smaller volume of sample than is required to flow around the entire capillary system to determine a test result.

In a capillary testing device, the first capillary pathway typically incorporates a reagent system capable of causing a reaction with a component of interest. Preferably, reagent may be deposited in a first test (assay) pathway. In the case of the arrangement described above, the reagent system is typically deposited in a first capillary pathway. Preferably, any test reagent is deposited upstream of an intersection with a second pathway.

Herein, inlets typically mean entry holes which are in fluid communication with a sample or fluid application region (i.e.. at the same end of the pathway), preferably in direct fluid communication. Thus, an inlet is the entry port for liquid into the pathway. If in indirect communication, this is preferably via non-capillary passages or means. An inlet is preferably provided at a proximal end of a pathway with an outlet at a distal end, although inlets may also be provided at one or more positions along the length of a pathway, for example for deposition of reagents in a passage or where branched (converging) channels or pathways are provided. An inlet must be of a dimension which enables it to receive liquid. Preferably, for a capillary testing device, an inlet will have an opening diameter in the region of 2 and 4mm, preferably between 1 and 2mm. For other applications, larger or smaller inlets are envisaged.

Typically, an outlet is provided to enable flow through a passage, for example by capillary of by a motive force, typically so that air can leave the passage. An outlet may be provided at a distal end of a pathway from an inlet (at the opposite end of the pathway from sample or fluid application regions) such that liquid flows toward it from the inlet. One or more outlets may be provided at one or more positions along the length of a pathway. An outlet may not need to accommodate liquid flow therethrough. Preferably, it is able to accommodate air flow therethrough, sufficient to maintain flow of a liquid through the respective pathway. For a capillary testing device, an outlet may be of smaller dimensions than an inlet. An outlet may typically have an opening diameter of between 0.5mm and 4mm, more preferably between 0.75 and 2mm. For other devices, larger or smaller outlets are possible. An outlet is typically only in fluid communication with a passage.

Outlets and inlets may have a raised skirt around circumference, with the outlet being central thereto.

A common outlet may lead to an outlet passage (which is conveniently a capillary pathway), and this may lead to a well, e.g. acting as a sump.

The first, second and third pathways may have the same or different internal architecture, e.g. in terms of cross-sectional area, cross-sectional profile etc. The form of the second pathway, for example its geometry and internal architecture will depend upon its purpose in the assay.

Each first, second or third pathway may have a constant or varying cross-section along its length.

In the present invention, a capillary pathway may have any suitable geometry, typically dictated by the array type. For instance, the passage may be straight, curved, serpentine, U-shaped, etc. The cross-sectional configuration of the capillary passage may be selected from a range of possible forms, e.g. triangular, trapezoidal, square, rectangular, circular, oval, U-shaped, etc. The capillary passage may have any suitable dimensions. Typical dimensions of a capillary passage for use in the invention is a depth of 0.1 mm to 1 mm, more preferably 0.2mm-0.7mm. The width of a channel may be of similar dimensions to the depth. Where the channel is V-shaped, for example, the profile may be that of an equilateral triangle, each side having a length of between 0.1 and 1 mm, more preferably between 0.2 and 0.7mm.

The liquid flow device of the invention conveniently forms part of a microfluidic system, i.e. a construct that facilitates a series of operations and manipulations involving liquids to be performed within an integrated device. Such devices generally comprise a series of fluidic features (such as channels, chambers, wells, etc) incorporated within or mounted on a substrate which provides support for the fluidic features.

The fluid flow control device is preferably applicable to any capillary pathway device, and finds application in a variety of microfluidic applications that require delivery or control of one or more liquids. Thus, it may be applicable to a microfluidic device, including for example lab-on-a-chip technology. The fluid flow control device may be provided in combination with devices which rely on other motive forces than capillary action to drive fluid flow, preferably as an integrated device. In such embodiments, reference to capillary action and capillary passages herein include within their scope any applicable fluid flow action or passage.

The invention is preferably used for sampling based assays, where a measured volume of liquid is removed from a larger volume and assayed. The present invention is particularly suited for use in assaying a sample liquid for a particular component. Whilst it may be suited to biological and non-biological applications, it is particularly suited to the former. Thus, the present invention is preferably for use in assaying a biological sample for a particular component, for example an analyte. Typically, assays for which the present invention may be used are microfluidics-based assays, including for example agglutination based assays, and coagulation based assays, in particular blood testing assays such as haematocrit assays. The assays are preferably quantitative. The present invention may be suitable for use with any liquid sample. Preferred biological samples for assay using the present invention are blood (whole blood or plasma) and urine.

The invention finds particular application in capillary testing devices having one or more capillary pathways for testing for the presence of a component of interest in a liquid sample, e.g. blood or other body fluid, as is well known in the art, e.g. diagnostic assays, such as the agglutination assays disclosed in WO 2004/083859 and WO 2006/046054.

The substrate can take a variety of formats, including but not limited to slides, discs, chips, etc. Any suitable material can be used for the substrate. Plastics, including but not limited to, polystyrene, poly(methyl methacrylate) (PMMA), acrylonitrile butadiene styrene (ABS), etc are frequently employed in microfluidic devices. If the substrate material is hydrophobic, it can be made hydrophilic (and thus capable of generating and permitting capillary flow) by a variety of means including, but not limited to, chemical treatments, coatings, plasma treatment etc. It is important to choose a means to promote hydrophilicity which does not interfere with any reactions that are carried out within the microfluidic device.

The fluidic features of the device can be produced in the substrate by a variety of means, including but not limited to moulding, embossing, machining, laser ablation, lithography, etc. Moulding or embossing are often preferred for volume production of uniform devices. Commonly the fluidic features are produced by formation of the features as indentations in the surface of the substrate, followed by application of a seal over the surface to form an enclosed system. Any suitable seal and sealing means can be employed to form the completed microfluidic features. In some embodiments the hydrophilic surface for the microfluidic system is brought from the seal. This can be obtained by using a seal material which is hydrophilic (or treated to be hydrophilic), or by using a hydrophilic adhesive to attach the seal.

In one embodiment, designed for use as a timing device, the first pathway comprises a test track and the second pathway comprises a timing track, with the pathways and liquid(s) being designed and selected so that liquid in the second pathway reaches the point of convergence after a desired, predetermined time that therefore determines the time in which liquid (typically a test sample e.g. blood sample, possibly followed by a chase buffer) flows in the first passage. This embodiment may, for example, be used to measure the viscosity of a test liquid by measuring the extent of flow in the first passage in the preset time. This may be employed, e.g., in an assay for measurement of haematocrit. The length of time of the test may be set by regulating the geometry and architecture of the second pathway, with the second pathway possibly being significantly longer (and of greater volume) than the first pathway, e.g. by having a portion of serpentine form, while the first pathway is of linear form or mainly linear form.

In another embodiment, designed to provide a reference control, the first pathway comprises a test track and a third pathway comprises a reference track (or control). The first and third pathways are preferably similar to each other in terms of track length, internal cross-section, architecture, etc., so as to have flow properties that are identical or substantially identical, e.g. being formed as mirror images of each other. The pathways conveniently have a common inlet, e.g. sample well, to which test liquid is supplied to pass to both pathways, possibly followed by a chase buffer. Such an embodiment may be used, for example, to provide a reference flow rate so that variations in endogenous sample viscosity do not affect results. This may be useful, e.g. in agglutination assays, with an agglutination reagent being provided in the first pathway and either a control reagent or no reagent being provided in the third pathway.

Another embodiment, as defined here, has first, second and third pathways, and can combine both the timing and reference functions of the embodiments discussed above. In this case, the device further comprises a third pathway having an inlet, with the first and third pathways intersecting at a downstream point of convergence to form a common outlet pathway, with the second pathway intersecting the common outlet pathway at a further point of convergence, and the first, second and third pathways all having a common outlet. The point of convergence and further point of convergence may be positioned very close to each other, effectively functioning as a single point of convergence. Alternatively, the first, second and third pathways may intersect with each other at two or more distinct points of convergence. All of these arrangements are functionally equivalent. The second pathway is typically used as a timing track to halt liquid flow in both the first and third pathways simultaneously after a predetermined time, determined by liquid flow in the second pathway reaching the further point of convergence, with the first and third pathway typically functioning as test and reference tracks, respectively, as discussed above.

In embodiments where it is required to measure the relative flow of liquid in the first and third pathways, e.g. where these function as test and reference tracks, the pathways conveniently include side-by-side, parallel, linear measurement portions, with the parts of the pathways upstream thereof being of corresponding form and having identical or substantially identical flow properties to each other.

The device may be in the form of a disposable device, intended for disposal after a single use.

A capillary testing device conveniently comprises a moulded plastics component, e.g. in the form of a generally planar element having grooves in one surface thereof to define the capillary passage(s) when sealed by a cover member.

The device preferably comprises a well, in fluid communication with (e.g. at the same end of a pathway as) a sample and/or fluid application region, which may comprise a sample application hole leading to a pathway. The well may be any suitable shape and size, suitable for receiving and retaining liquid sample. The well may be provided (in full or in part) by the device, by the fluid flow control device or by fluid dispensing means. A well may comprise features, for example micropillars, to aid sample liquid flow into a capillary passage. Suitable features will be known to a person skilled in the art.

In embodiments of the invention, for example where capillary action is used to move liquid sample in the passages, fluid dispensing means may be provided. Preferably, fluid dispensing means comprise a rupturable, sealed container of fluid to be dispensed, rupturing means for rupturing the container and releasing the contents, the container and rupturing means being arranged for relative movement between a first position in which the container is intact and a second position in which the container is ruptured. The dispensing means can enable fluid to be dispensed reliably in known quantities, determined by the container contents, even small volumes such as 1000 microlitres or less, 500 microlitres or even less.

A device of the invention can thus be easy to operate, to deliver a predetermined volume of fluid, and can be used reliably by relatively unskilled personnel.

One or more detection regions may be provided in a capillary pathway, for example to aid measurement of the distance travelled or the position of the fluid front in a pathway. Detections means may comprise a window.

A capillary pathway of the device may be treated to improve flow of liquid sample therethrough, by passing treatment fluid through the passage to leave a surface coating on the internal surface of the passage. Thus, a capillary pathway of the device may comprise a coating on the inner surface thereof, of a treatment fluid.

The coating typically acts by minimising any repulsion between the inner surface of the passage and sample fluid, whilst preferably not actively binding or substantially reacting with any sample, fluid or component thereof. Preferably, the surface coating increases the hydrophilicity of the passage, as compared to an untreated passage. The coating may, for example, act by forming a layer on the inner surface of the treated passage, polymerising with the surface of the treated passage, or soaking into the material of the treated passage.

The treatment fluid may be a liquid or a gas, but typically is a liquid, preferably having suitable hydrophilic properties, e.g. a surfactants. Suitable materials are well known to those skilled in the art, and include for example polysorbates, commonly being used for this purpose, particularly polyoxyethylene sorbitan materials known as Tween (Tween is a Trade Mark), e.g. Tween 20 (polyoxyethylene (20) sorbitan monolaurate), Tween 60 (polyoxyethylene (20) sorbitan monostearate), Tween 80 (polyoxyethylene (20) sorbitan monooleate). Such materials are typically used in the form of dilute aqueous solutions, e.g 0.1 to 10%, typically. 1 % by volume or less, typically in deionised water, although other solvents such as isopropanol (IPA) may alternatively be used.

The present invention provides a capillary pathway device, as described herein.

### Brief Description of the drawings

The invention will now be described, by way of illustration, with reference to the accompanying drawings, in which:
Figure 1 is a schematic diagram illustrating one embodiment of a liquid flow device in accordance with the invention;
Figure 2 is a schematic diagram illustrating a further embodiment of a liquid flow device in accordance with the invention;
Figure 3 is a schematic diagram illustrating yet a further embodiment of a liquid flow device in accordance with the invention;
Figure 3A shows to an enlarged scale part of the embodiment of Figure 3; and
Figure 4 shows to an enlarged scale part of a variant of the embodiment of Figures 3 and 3A.
Figure 5 is a schematic diagram illustrating a further variant of the embodiment of Figures 3 and 3A; and
Figure 6 is a schematic sectional view of the arrangement of Figure 5.
Figure 7 shows the relationship between hct and time to reach the end of lane 3 (280 mm from the start of the capillary) for a variety of samples.
Figure 8 shows the relationship between distance travelled and time elapsed for 30-50% hct samples and PBS chase buffer.
Figure 9 shows the relationship between distance measured using the prototype test device and method of the current invention and haematocrit of a series of whole blood samples determined by a standard centrifguation method (Hettich, Haematokrit 210).
Figure 10 shows the flow profiles for the 0% and 25% D-dimer test channels, control channels and PBS.
Figure 11 is a schematic diagram showing a variant of a device of the invention with a first and second pathway.

### Detailed description of the drawings

The drawings illustrate schematically (and not to scale) embodiments of liquid flow devices in accordance with the invention in the form of capillary flow test devices, constituting examples of microfluidic devices for use in testing liquid samples, commonly a body fluid such as blood (whole blood or plasma), urine, saliva, cerebrospinal fluid, etc., for the presence of a component of interest (qualitatively or quantitatively).

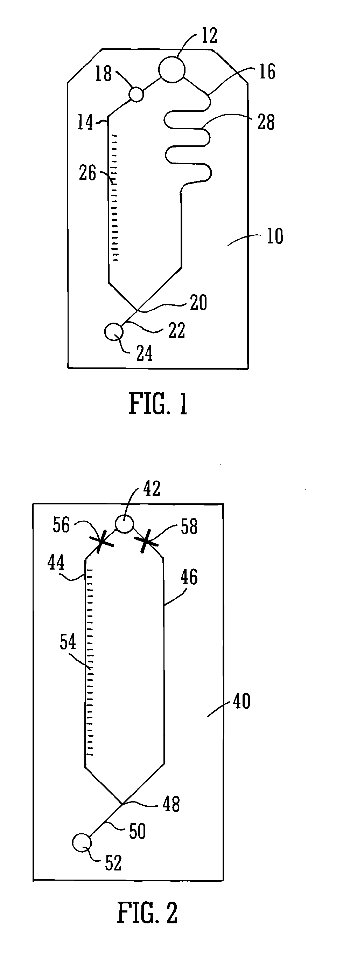

Figure 1 illustrates a device comprising a generally rectangular planar member 10 having formations defining connected wells and passages constituting an enclosed system. These include a reagent well 12 from which extend a first capillary passage 14 and a second capillary passage 16. A sample port 18 is provided in the first passage 14, spaced slightly downstream from the well 12. The passages 14 and 16 merge or intersect downstream at a point of convergence 20, to form a common downstream or distal outlet capillary passage 22 leading to a well 24 constituting a sump. The outlet passage 22 and well 24 together constitute a common outlet for the first and second passages. The first passage 14 includes a major linear portion, with a series of distance measurement markings 26 constituting a measurement gauge provided along the edge of this portion. The second passage 16 includes a serpentine portion 28, so that the second passage 16 (extending from the well 12 to the point of convergence 20) is longer than the first passage 14 (extending from the well 12 to the point of convergence 20).

The element 10 is suitably of rigid transparent plastics material such as polycarbonate, with the wells and passages etc. conveniently being formed by injection moulding.

The device can be used to control the length of time that liquid flow occurs in the first passage 14, with the first passage constituting a test track and the second passage 16 constituting a timing track.

To perform a test, a known volume of test liquid, such as a blood sample, is added to the device via sample port 18. The liquid will flow along the first passage 14 in both directions. The liquid will only flow upstream as far as the reagent well 12; capillary force will keep the liquid within the passage 14 and will not permit the liquid to flow into the well 12. The liquid will flow in a downstream direction, towards the point of convergence 20, by capillary force, producing a static column of test liquid of known volume in the first capillary passage 14.

To initiate a test, a reagent, typically a chase buffer, is added to the device via the reagent well 12. The reagent flows along both the first and second capillary passages by capillary force, pushing the test liquid ahead of it in the first passage (test track). The rate of flow in each of the two passages depends on the viscosity of the liquid (test liquid or reagent), the cross-sectional area of the passage and also the geometry of the passage. For example, capillary flow slows down when going around corners of sufficient curvature.

Liquid will continue to flow along the two passages 14 and 16 until liquid in one of the passages reaches the point of convergence 20. The design of the device (in terms of the length, cross-sectional area and architecture of the first and second passages) and the selection of chase buffer or other reagent (in terms of viscosity in relation to the viscosity of the test liquid) are such that it is the liquid in the second passage 16 (the timing track) that reaches the point of convergence 20. This causes liquid flow in the first passage 14 (the test track) to stop, as the downstream escape of air from the passage (via the outlet passage 22 and the sump 24) is now prevented. Liquid can continue to flow in the second passage 16 along the outlet passage 22 to be collected in the sump 24.

The extent of liquid flow in the test track can be determined by use of the measurement gauge 26. This can be done by eye, on a simple visual inspection, or by use of suitable instrumentation.

The device and test can be tailored to produce a desired test duration of liquid flow in the test track.

The device of the invention can thus be used to control the time of a test. For example, this may be used to measure the viscosity of a test liquid.

One application is thus in an assay for measurement of haematocrit. The viscosity of a blood sample is largely dependent on the concentration of erythrocytes, the higher the concentration the higher the viscosity and thus the shorter the distance the blood will travel in a given time. A sample of known volume of anticoagulated blood is added to a device via the sample port 18. Alternatively, whole blood may be added, with an anti-coagulant provided in the port 18. A chase buffer, of lower viscosity than whole blood, is then added to the device via well 12, with liquid flow occurring as described above, to allow flow in the test track for a predetermined time. The distance that the test blood sample travels in this time will depend on the viscosity of the blood sample (and thus the haematocrit concentration). By determining the distance travelled by the test blood sample in the test track by reference to the suitably calibrated measurement markings 26, the haematocrit level can be determined.

The device is thus very simple and easy to use. Measurements can be made by eye, without requiring any instrumentation or readers. No power sources or operator intervention are required to control fluid flow.

A device as described typically comprises an element with dimensions of about 100 mm x 50 mm, with capillary passages having a maximum cross-sectional dimension of about 0.5 mm. This is suitable for use with a small sample volume, e.g. about 10 to 15 microlitres, as can be collected using a fingerstick device.

The device is intended for single use, being disposed of after use.

Figure 2 illustrates a further embodiment of test device that is generally similar to the device of Figure 1. The Figure 2 device comprises a generally rectangular planar member 40 having formations defining connected wells and passages. These include a sample/reagent well 42 from which extend a first capillary passage 44 (test track) and a second capillary passage 46 (reference track) that merge downstream at a point of convergence 48 to form an outlet passage 50 leading to a well 52 that constitutes a sump. The first and second passages are formed as mirror images of each other, having the same path direction and length and having the same cross-sectional profiles so that the two passages have the same length and volume. A series of distance measurement markings 54 constituting a measurement gauge are provided along the length of part of the first capillary passage 44.

The device can be used to provide a reference and so compensate for any variation in sample viscosity. This can be of assistance in certain diagnostic assays, such as agglutination assays e.g. as disclosed in WO 2004/083859 and WO 2006/046054. In this case the first passage 44 (test track) incorporates a reagent system deposited within the passage represented schematically at 56 capable of causing an agglutination reaction with a component of interest in a sample. The second passage 46 (reference track) incorporates a control reagent deposited within the passage represented schematically at 58 which does not cause an agglutination reaction (or possibly no reagent is present in the reference track).

In use of the device, a known volume of test liquid, such as a blood sample, is added to the device via the well 42. The liquid will flow along both the first and second passages, past reagents 56 and 58, by capillary force. This is typically followed by addition of a chase buffer.

If the component of interest is present in the sample, agglutination will occur in the test track but not in the reference track, with the degree of agglutination depending on the concentration of the component in the sample. Agglutination increases the viscosity of the test liquid and slows the rate of flow in the test track in a manner which relates to the concentration of the component. When the faster flowing liquid in the reference track reaches the point of convergence 48, the flow in the test track stops. The time for the test liquid to reach the point of convergence depends, *inter alia,* on the endogenous viscosity of the sample. The distance travelled by the test liquid in the test track in this time depends, *inter alia,* on the endogenous viscosity of the liquid and the increase in viscosity due to agglutination. However, since the reference track effectively corrects for endogenous viscosity, the distance travelled in the test track is indicative of the concentration of the component of interest in the sample liquid. This can be determined using the suitably calibrated measurement markings 54.

Once the fluid flow has stopped, the end point in the test track will remain constant so there is no requirement for a user to monitor fluid flow continually throughout the assay (as would be the case with measurement based on timings), and no instrumentation or power sources are required.

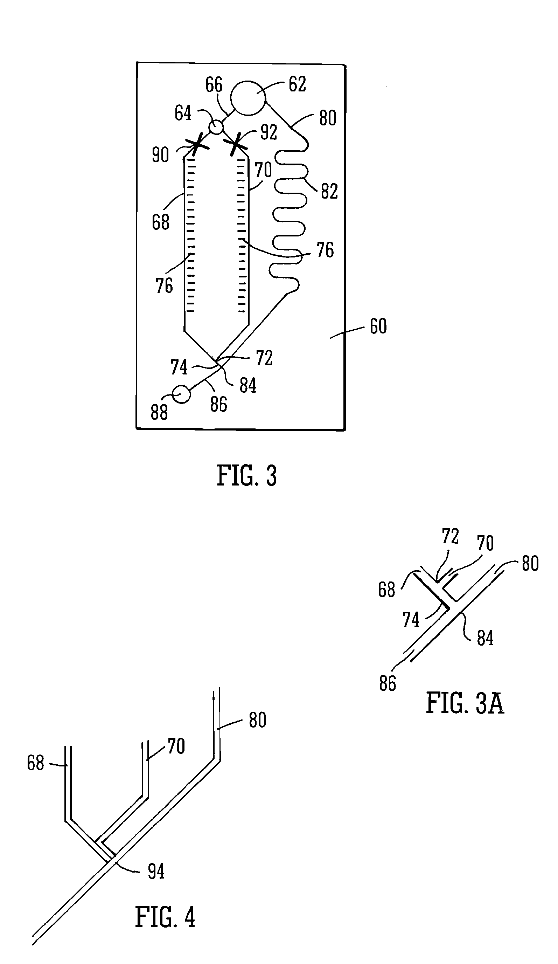

Figure 3 illustrates a further embodiment of test device that effectively combines the features of the embodiments of Figures 1 and 2.

The Figure 3 device comprises a generally rectangular planar member 60 having formations defining connected wells and passages. These include a first well 62 constituting a primary chamber for receiving reagent and a second, smaller well 64 constituting a secondary chamber for receiving sample, linked by a first capillary passage 66. A second capillary passage 68 and a third capillary passage 70, formed as mirror images of each other, extend from the well 64, the passages merging at a first point of convergence 72 to form a fourth capillary passage 74 constituting a common downstream or distal outlet passage. A series of distance measurement markings 76, 78 constituting measurement gauges are provided along the length of the passages 68, 70, respectively. A fifth capillary passage 80 extends from the first well 62, having a serpentine portion 82, and merging with the fourth passage at a second point of convergence 84 to form a sixth capillary passage 86 constituting a common downstream or distal outlet, leading to a well 88 constituting a sump.

Passage 68 constitutes a test track (and incorporates a test reagent represented at 90) and passage 70 constitutes a reference track (and incorporates a control reagent represented at 92, or no reagent), as in the Figure 2 embodiment, e.g. allowing for compensation of variations in sample viscosity. Passage 80 constitutes a timing track, as in the Figure 1 embodiment, allowing control of the length of time that liquid flow occurs in both the test track 68 and reference track 70.

In use of the device, a known volume of test liquid, such as a blood sample, is added to the sample well 64, followed by addition of a reagent, e.g. a chase buffer, to well 62. Fluid flow in the timing track 80 depends on the viscosity of the reagent, and the time taken to reach the second point of convergence 84 (which determines the overall assay time) depends on the length and geometry of the channel 80 as well as the viscosity. The sample flow rate in the reference track depends on the endogenous viscosity of the sample, while the sample flow rate in the test track depends on the endogenous viscosity and the change of viscosity brought about by reaction with test reagent 90, typically an agglutination reagent. By comparing the distance travelled in the test track and reference track in the time determined by the timing track, by use of the measurement markings 76 and 78, information can be obtained about a component of interest in the sample liquid.

This embodiment finds use in cases where it is necessary to control both the exact time for the assay and compensate for any variation in sample viscosity. For example, in coagulation assays it is possible to determine the time for sample coagulation to occur by observing how far a sample can migrate along a capillary before coagulation stops fluid flow. However, the absolute distance traversed will depend on both the time for coagulation to occur and the endogenous viscosity of the sample (especially when attempting to determine the coagulation time for whole blood). Control of both time and viscosity can be achieved by using a 3 channel device, as in Figure 3. The test track contains a coagulation or clotting reagent 90, whilst the reference track remains empty or contains a control reagent 92 which will not cause coagulation.

Figure 4 illustrates a variant of the embodiment of Figures 3 and 3A, where the first point of convergence 72 and the second point of convergence 84 effectively coincide, with passages 68 and 70 both merge with passage 80 at substantially the same point 94. This variant does not affect functioning.

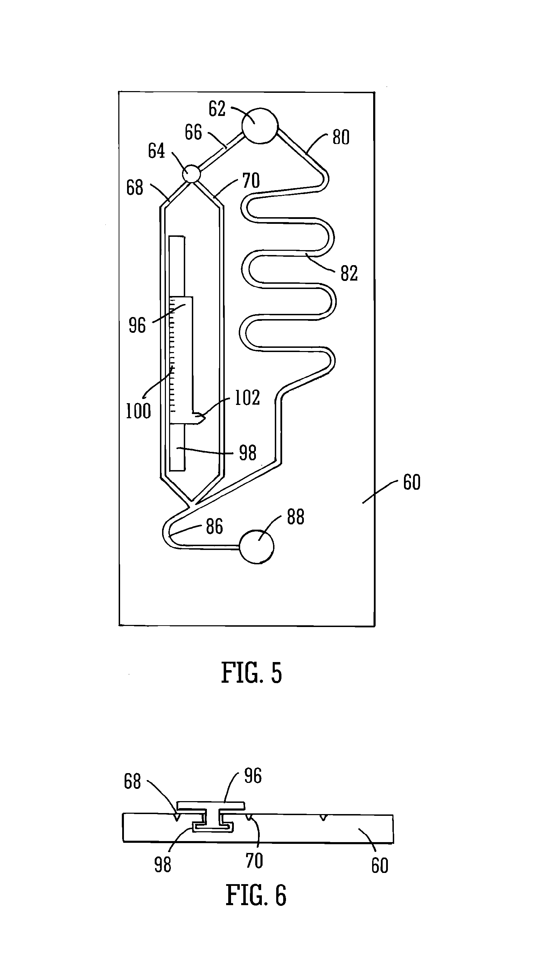

Figures 5 and 6 illustrate a further variant of the embodiment of Figures 3 and 3A, with corresponding items having the same reference numbers. In place of distance measurement markings 76 and 78, this embodiment includes elongate cursor 96 slidably received in an elongate groove 98 formed in the upper surface of the planar member 60 located between the capillary passage 68 (test track) and the capillary passage 70 (reference track) and extending parallel thereto. Measurement markings 100 are provided along the edge of the cursor, adjacent the test track 68, with a pointer 102 extending from the downstream end of the cursor towards the reference track 70.

The device is used as described above in connection with Figures 3 and 3A. After liquid flow in passages 68 and 70 has stopped, as determined by flow in the timing passage 80, the cursor is positioned with the pointer 102 aligned with the leading edge of liquid flow in reference track 70. The position of liquid in the test track 68 can then be determined by reference to the measurement markings 100. The measurement markings may represent distance, which can then be used to determine concentration of a component of interest in a sample. Alternatively, the markings may represent analyte concentration, determined by means of a predetermined dose response, enabling analyte concentration to be read directly. The sliding cursor enables analyte concentration to be determined very simply and accurately with little risk of error.

The sliding cursor arrangement may be modified in various ways, for instance with the measurement markings being provided along the edge of the cursor adjacent the reference track 70 and with a pointer (or equivalent) on the opposite edge of the cursor at or near the upstream end thereof.

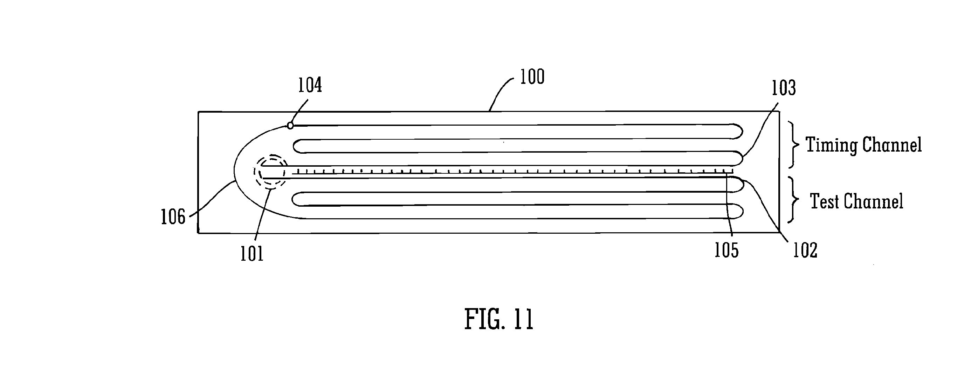

Figure 11 illustrates a device comprising a generally rectangular planar member 100 having formations defining connected wells and passages constituting an enclosed system. These include a loading port 101 from which extend a first capillary passage 102 and a second capillary passage 103. The passages 102 and 103 merge or intersect downstream at a point of convergence 104. A series of distance measurement markings 105 constituting a measurement gauge provided along the edge of this portion. The first passage 102 includes an extra portion 106, so that the first passage 106 is longer than the second passage 102.

The element 100 is suitably of rigid transparent plastics material such as polycarbonate, with the wells and passages etc. conveniently being formed by injection moulding.

The device can be used to control the length of time that liquid flow occurs in the first passage 102, with the first passage constituting a test track and the second passage 103 constituting a timing track.

To perform a test, a known volume of test liquid, such as a blood sample, is added to the device via port 101. The liquid will flow along the first passage 102. The liquid will flow in a downstream direction, towards the point of convergence 104, by capillary force, producing a static column of test liquid of known volume in the first capillary passage 102.

To initiate a test, a reagent, typically a chase buffer, is added to the device. The reagent flows along both the first and second capillary passages by capillary force, pushing the test liquid ahead of it in the first passage (test track). The rate of flow in each of the two passages depends on the viscosity of the liquid (test liquid or reagent), the cross-sectional area of the passage and also the geometry of the passage. For example, capillary flow slows down when going around corners of sufficient curvature.

Liquid will continue to flow along the two passages 102 and 103 until liquid in one of the passages reaches the point of convergence 104. The design of the device (in terms of the length, cross-sectional area and architecture of the first and second passages) and the selection of chase buffer or other reagent (in terms of viscosity in relation to the viscosity of the test liquid) are such that it is the liquid in the second passage 103 (the timing track) that reaches the point of convergence 1040. This causes liquid flow in the first passage 102 (the test track) to stop, as the downstream escape of air from the exhaust port 104 is now prevented.

The extent of liquid flow in the test track can be determined by use of the measurement gauge 105. This can be done by eye, on a simple visual inspection, or by use of suitable instrumentation.

### Examples

The following Examples 1-3 describe development of a test and device using the invention to quantitate the haematocrit (red blood cell concentration by % volume) of samples of whole blood. Examples 4 and 5 describe development of a test and device using the invention to quantitate the D-dimer analyte (cross-linked fibrin degradation products) in whole blood samples using an autologous agglutination immunoassay.

### Example 1

Initially, the relationship between haematocrit (hct) and flow rate was established. An experimental test device comprising a planar chip moulded from polycarbonate containing two serpentine capillary tracks was used for these studies. The capillary tracks had a V-shaped cross-section (535 um wide), each with a total length of 460 mm arranged as 5 parallel straight sections joined by U-shaped curves. At the proximal end was a loading port for addition of fluids.

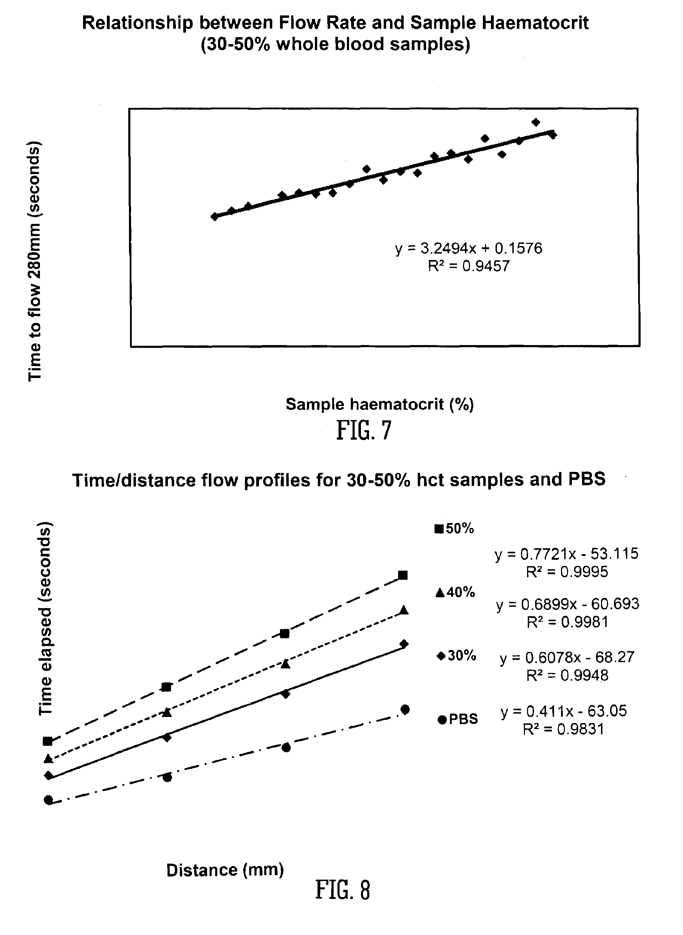

The haematocrit of anticoagulated whole blood samples (citrate phosphate dextrose anticoagulant) were adjusted to cover the range from 30%-50%, and a 20uL sample of each was added to the loading port, followed by 300uL of chase buffer (phosphate buffered saline pH 7.4, PBS). The time for the fluid front to reach the end of each lane was recorded. Figure 7 shows the relationship between hct and time to reach the end of lane 3 (280 mm from the start of the capillary) for a variety of samples. As can be seen, between 30% and 50% there is a clear linear relationship between time and distance (R² = 0.95).

### Example 2

To determine the dimensions of a suitable test device, the data from example 1 was processed to compare the distance travelled against time elapsed by samples of differing haematocrit. In addition, the distance travelled by chase buffer in the absence of sample was also plotted, to allow the length of the timing channel to be determined. Figure 8 shows the relationship between distance travelled and time elapsed for 30-50% hct samples and PBS chase buffer. Table A shows the distances reached by the sample front at 90 and 120 seconds, calculated using the linear regression equations shown in Figure 8.

**Table A**

| Distance reached by fluid front of 30-50% hct samples and PBS at 90 and 120 seconds | | |
|---|---|---|
| Sample haematocrit | Distance at 90 seconds (mm) | Distance at 120 seconds (mm) |
| 30% | 260 | 310 |
| 40% | 218 | 262 |
| 50% | 185 | 224 |
| PBS chase buffer | 372 | 445 |

### Example 3

A reusable prototype test device was constructed to allow whole blood haematocrit determinations to be performed. This comprised a planar polycarbonate chip containing 2 serpentine capillary channels; a test channel and a timing channel. The capillaries have a V-shaped cross section 535 um wide, each originating at a common fluid loading port and converging at a common exhaust outlet. The timing capillary was 370 mm long, in the region from the buffer addition port to the point of channel convergence. The test capillary incorporated a series of gradations alongside the track, spanning the distance 150-350 mm from the origin (based on distances determined in example 2). The gradations were used to determine the stopping distances of a range of samples of known hct.

To use the device, 10uL of whole blood was added directly into the test capillary via the loading port. When the entire sample had entered the test capillary, chase buffer (200uL of PBS) was added via the same port, causing fluid to flow along both test and timing capillaries. When fluid in the timing capillary reached the point of convergence (exhaust outlet) no further flow occurred in the test capillary. The position reached by the fluid front in the test capillary was read, using the gradations alongside.

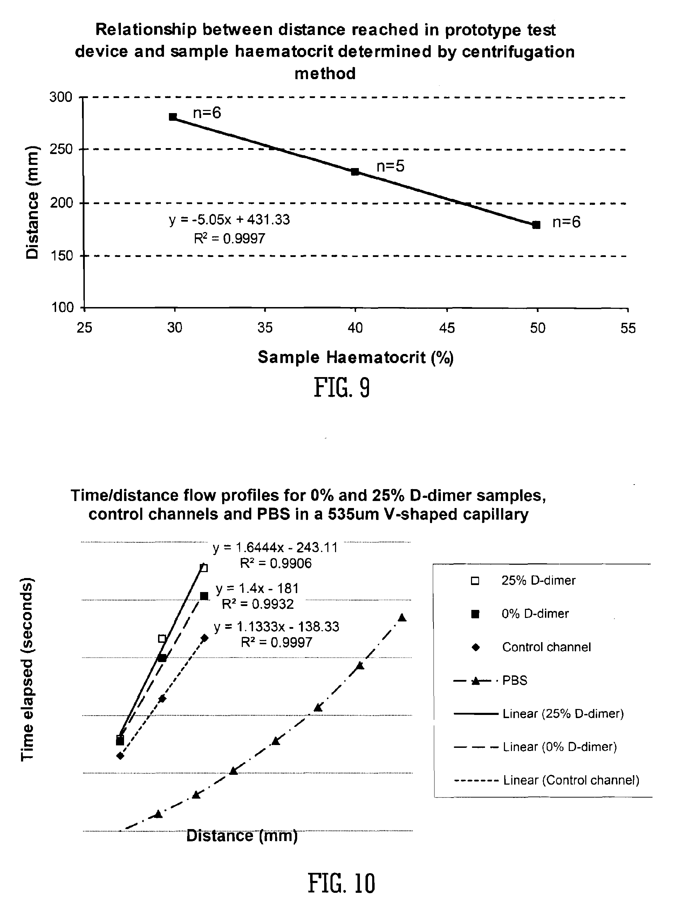

Table B shows the distances travelled by the fluid in the test lane before flow was stopped by fluid in the timing channel reaching the point of convergence, and Figure 9 shows the relationship between distance measured using the prototype test device and method of the current invention and haematocrit of a series of whole blood samples determined by a standard centrifguation method (Hettich, Haematokrit 210).

**Table B**

| Distance reached by 30-50% hct whole blood samples in prototype test device | |
|---|---|
| Sample haematocrit (%) | Mean distance reached (mm) |
| 30 | 280.3 (n=6) |
| 40 | 228.4 (n=5) |
| 50 | 179.3 (n=6) |

### Example 4

An experimental test device comprising a planar chip moulded from polycarbonate containing two serpentine capillary tracks was used. The capillary tracks had a V-shaped cross-section (535 um wide), each with a total length of 460 mm arranged as 5 parallel straight sections joined by U-shaped curves. At the proximal end was a loading port for addition of fluids.

A commercial autologous agglutination reagent (7.5ul SimpliRED D-dimer Test Reagent, BBI) was deposited into lane 4 of one of the capillary tracks and allowed to air-dry in a desiccated chamber. The capillary containing desiccated reagent was designated "test channel", whilst the second capillary, without agglutination reagent, was designated "control channel".

Blood samples were prepared by removing an equal volume of plasma from each sample and replacing with D-dimer Positive Control Reagent (SimpliRED D-dimer, BBI) or saline/BSA solution (0.9% NaCl, 0.5% BSA, Sigma). Samples generated in this manner contained "0%" and "25%" D-dimer with respect to the concentration of the SimpliRED D-dimer kit Positive Control Reagent.

To run an assay, 30ul of sample was introduced into the loading port of the device and allowed to enter the two capillaries fully before 500ul of chase buffer (phosphatebuffered saline, PBS) was added to the loading port. The time was recorded for the fluid in both channels to reach the end of each lane and the end of the 460mm track.

A second experimental test device was created with a single 880mm-long serpentine capillary, arranged as 10 parallel straight sections (lanes) joined by U-shaped curves. The capillaries contained within the second device and the twin-channel device described above were of identical interior dimensions other than length. A loading port was located at one end of the capillary.

To run a sample, 500ul PBS was added to the loading port and times were recorded for the fluid to reach the end of each of the 10 lanes and the end of the 900mm track.

Figure 10 shows the flow profiles for the 0% and 25% D-dimer test channels, control channels and PBS. Table C shows the distances reached in 535um V-shaped capillary by 0% and 25% D-dimer samples, control channel and PBS after 350 seconds have elapsed, using the flow profile data displayed in Figure 10.

**Table C**

| Mean distance reached by 0% and 25% D-dimer samples, Control channel and PBS after 350 seconds | |
|---|---|
| 0% D-dimer sample | 379.3mm (n=6) |
| 25% D-dimer sample | 360.7mm (n=6) |
| Control channel | 430.9mm (n=12) |
| PBS | Approx 800mm (n=3) |

### Example 5

A reusable prototype test device was constructed to allow D-dimer concentrations to be determined in whole blood. This comprised a planar polycarbonate chip containing 2 serpentine capillary channels; a test channel and a timing (control) channel. The capillaries have a V-shaped cross section 535 um wide, each originating at a common fluid loading port and converging at a common exhaust outlet. The timing capillary was 370 mm long, in the region from the buffer addition port to the point of channel convergence. The test capillary incorporated a series of gradations alongside the track, spanning the distance 280-370 mm from the origin. The gradations were used to determine the stopping distances of samples containing known concentrations of D-dimer analyte.

To prepare devices for performing D-dimer assays, a commercial autologous agglutination reagent (7.5ul SimpliRED D-dimer Test Reagent, BBI) was deposited in lanes 1 and 2 of one of the capillary tracks and allowed to air-dry in a desiccated chamber. The capillary containing desiccated reagent was designated "test channel", whilst the second capillary, without agglutination reagent, was designated "timing (control) channel".

Blood samples were prepared by removing an equal volume of plasma from identical aliquots of whole blood and replacing with D-dimer Positive Control Reagent (SimpliRED D-dimer, BBI) or saline/BSA solution (0.9% NaCl, 0.5% BSA, Sigma). Samples generated in this manner contained "0%" and "25%" D-dimer with respect to the concentration of the SimpliRED D-dimer kit Positive Control Reagent.

To use the device, 15uL of "control" whole blood was added directly into the control capillary via the loading port and 15ul of "test sample" was added directly to the test capillary. A delay of 90 seconds was allowed to elapse before chase buffer (200uL of PBS) was added via the same port, causing fluid to flow along both test and timing (control) capillaries. When fluid in the timing capillary reached the point of convergence (exhaust outlet) flow stopped in the test capillary. The position reached by the fluid front in the test capillary was read, using the gradations alongside.

Table D shows the mean distances travelled in the test lane of the prototype device for blood samples containing 0% and 25% D-dimer (see above) before flow stopped due to fluid in the timing channel reaching the point of channel convergence.

**Table D**

| Test blood sample D-dimer concentration | Mean distance travelled by sample in test channel (stopping distance) | Mean time for timing (control) channel to reach convergence (stopping time) |
|---|---|---|
| 0% | 347 mm (n=3) | 238 seconds (n=6) |
| 25% | 298 mm (n=3) | |

The invention further includes the subject matter of the claims of WO 2012164263 from which this application is derived, the content of which is reproduced below as numbered paragraphs.
1. A liquid flow device comprising a first pathway having an inlet, and a second pathway having an inlet, the first and second pathways intersecting at a downstream point of convergence and having a common outlet, and means for measuring the extent of liquid flow along the first pathway.
2. A liquid flow device for quantitative measurement of a substance of interest in a liquid according to paragraph 1, wherein the liquid flow device is a capillary testing device, comprising a first capillary pathway having an inlet and a second capillary pathway having an inlet, the first and second pathways intersecting at a downstream point of convergence and having a common outlet, and means for measuring the extent of liquid flow along the first capillary pathway.
3. A liquid flow device according to paragraph 1 or paragraph 2, wherein measurement means comprise distance markings provided in relation to at least the first pathway, to measure the distance liquid has travelled.
4. A liquid flow device according to paragraph 3 wherein the distance markings are provided along a first pathway, substantially from the inlet to a point of convergence with a second pathway.
5. A liquid flow device according to any one of paragraphs 1 to 4, wherein the measurement means comprise an elongate cursor, having distance markings provided along at least one elongate edge thereof, and positioned parallel to at least part of the length of a first pathway.
6. A liquid flow device according to paragraph 5 wherein the elongate cursor is slidable with respect to a first pathway.
7. A liquid flow device according to paragraph 5 or 6, wherein the elongate cursor is provided with one or more indicators for alignment with the leading edge of liquid in a pathway.
8. A liquid flow device according to any one of paragraphs 5 to 7 wherein the cursor is provided in a groove of the liquid flow device, and preferably is slidable within the groove.
9. A liquid flow device according to any one of the preceding paragraphs comprising a third pathway which functions as a reference pathway, for normalising variation between samples.
10. A liquid flow device according to paragraph 9 wherein the third pathway shares a common inlet with the first pathway.
11. A liquid flow device according to any one of paragraphs 9 or 10 wherein the third pathway intersects the first pathway at a downstream point of convergence.
12. A liquid flow device according to paragraph 11 wherein the second pathway intersects the first pathway a point of convergence downstream to the point of convergence of the first and third pathways, so that liquid flow in the first and third pathways can be stopped simultaneously when liquid in the second pathway reaches the point on convergence with the first pathway.
13. A liquid flow device according to any one of paragraphs 9 to 12 wherein measurement means are provided in relation to the third pathway.
14. A liquid flow device according to paragraph 13 wherein the measurement means are shared between a first pathway and a third pathway and arranged for measuring the relative difference in distance travelled between liquids in the first pathway and the third pathway.
15. A liquid flow device according to paragraph 14 wherein the measurement means as defined in any one of paragraphs 4 to 8.
16. A liquid flow device according to paragraph 15 wherein the cursor is provided between linear portions of the two or more pathways.
17. A liquid flow device according to any one of paragraphs 9 to 16, wherein the first pathway and third pathway are substantially identical in terms of geometry and architecture.
18. A liquid flow device according to any one of the preceding paragraphs, comprising two or more first pathways, and measurement means provided for determining the extent of liquid flow in each.
19. A liquid flow device according to any one of the preceding paragraphs, comprising reagent, e.g. test or control reagent, in one or more pathways.
20. A liquid flow device according to any one of the preceding paragraphs wherein pathway is straight, curved, serpentine, or U-shaped, or a combination thereof; and wherein the cross-sectional configuration of a pathway is selected from triangular, trapezoidal, square, rectangular, circular, oval, or U-shaped.
21. A liquid flow device according to any one of the preceding paragraphs, comprising metering means to control liquid volume supply for each pathway.
22. A liquid flow device according to any one of the preceding paragraphs which is a chip.
23. A liquid flow device according to any one of the preceding paragraphs comprising a well, in fluid communication with a sample and/or fluid application region.
24. A liquid flow device according to any one of the preceding paragraphs comprising fluid dispensing means.
25. A liquid flow device according to any one of the preceding paragraphs comprising one or more detection regions in a pathway.
26. A liquid flow device according to any one of the preceding paragraphs, wherein at least one pathway is treated to improve flow of liquid sample therethrough.
27. A kit for quantitative measurement of a substance of interest in a liquid comprising a liquid flow device according to any one of the preceding paragraphs, in combination with a chart correlating extent of liquid travel in a first pathway with amount of substance of interest present in the liquid; and optionally buffers and/or reagents.
28. A method of measuring the extent of liquid flow along a first pathway that is intersected by a second pathway at a downstream point of convergence, with the first and second pathways having a common outlet, comprising causing or permitting liquid to flow in the first pathway and causing or permitting liquid to flow in the second pathway at least until it reaches the point of convergence, and measuring the extent to liquid flow in the first pathway once the liquid in the second pathway has reached the point of convergence.
29. A method of measuring the extent of liquid flow along a first pathway of a liquid flow device according to paragraph 28, where the first pathway extends from a sample application region to an outlet and is intersected at a downstream point of convergence by a second pathway which extends from a fluid application region, such that the first and second pathways share a common outlet, wherein the method comprises causing or permitting liquid to flow in the first pathway and causing or permitting liquid to flow in the second pathway at least until it reaches the point of convergence, and measuring the distance travelled by liquid in the first pathway once the liquid in the second pathway has reached the point of convergence.
30. A method of measuring the extent of liquid flow along a first pathway of a liquid flow device according to paragraph 28:
   i) providing a liquid flow device comprising a first pathway having an inlet and a second pathway having an inlet, the first and second pathways intersecting at a downstream point of convergence and having a common outlet, and means for measuring the extent of liquid flow in a first pathway;
   ii) causing or permitting liquid to flow in the first and second pathways from respective inlets toward the outlet;
   iii) using the measuring means of the device to determine the distance travelled by liquid along the first pathway, preferably once liquid in the second pathway has reached the point of convergence.
31. A method according to any one of paragraphs 28 to 30 for quantitative measurement of a substance of interest in a liquid, preferably further comprising the step of correlating the distance travelled by liquid in the first pathway to the amount of substance of interest in the liquid (sample) of the first pathway, by using a chart correlating distance and amount for known samples.
32. A method according to any one of paragraphs 28 to 31, for measuring the amount of a substance of interest in a liquid, preferably wherein the substance is haematocrit.
33. A method for performing a timed assay according to any one of paragraphs 28 to 32, comprising
   i) providing a liquid flow device wherein the length of time of the test is set by regulating the geometry and architecture of the second pathway;
   ii) causing or permitting liquid to flow in a first pathway and a second pathway;
   iii) measuring the extent of flow in the first pathway in the preset time determined by the time taken for liquid in the second pathway to reach the point of convergence with the first pathway.
34. A method according to paragraph 33, wherein the device comprises a third pathway, and ii) comprises causing or permitting liquid to flow in a first, second and third pathways; and ii) comprises determining the difference in extent of liquid travel in the first and third pathways; iii) correlating the distance travelled with the amount of substance on interest in the liquid.
35. A method according to any one of paragraphs 28 to 34 wherein the device is as defined in any one of paragraphs 1 to 26.

## Claims

1. A microfluidic liquid flow device comprising i) a test pathway having an inlet, and ii) a timing pathway having an inlet, the test and timing pathways intersecting at a downstream point of convergence and having a common outlet, and iii) means for measuring the extent of liquid flow along the test pathway;
wherein the timing pathway is longer than the first pathway and sets a predetermined time period during which liquid can flow in the test pathway.

2. A microfluidic device according to claim 1 wherein a portion of the timing pathway is of serpentine configuration.

3. A microfluidic device according to claim 2 wherein the test pathway is linear.

4. A microfluidic liquid flow device according to any one of claims 1 to 3 comprising a common inlet from which extends a test pathway comprising a sample port downstream of the common inlet and a timing pathway, the test and timing pathways intersecting at a downstream point of convergence and having a common outlet, and means for measuring the extent of liquid flow along the first pathway.

5. A microfluidic liquid flow device according to claim 4 comprising a reference pathway, wherein the reference pathway extends from the sample port of the test pathway.

6. A microfluidic liquid flow device according to claim 5 wherein the reference pathway intersects the test pathway at a downstream point of convergence and shares a common outlet.

7. A microfluidic liquid flow device according to claim 5 or 6 wherein the reference pathway intersects the test pathway upstream of the intersection with the timing pathway, such that liquid flow in the test and reference pathways is stopped simultaneously when liquid in the timing pathway reaches the intersection with the test pathway.

8. A microfluidic liquid flow device according to any one of claims 1 to 7, comprising distance markings provided in relation to the test pathway and optionally the reference pathway, to measure the distance liquid has travelled.

9. A microfluidic liquid flow device according to claim 8, wherein the test and reference pathways comprise parallel linear measurement portions, and wherein the test and reference pathways upstream of the measurement portions have substantially identical form and flow properties.

10. A microfluidic liquid flow device according to any one of the previous claims, comprising an elongate cursor having distance markings provided along at least one elongate edge thereof and positioned parallel to at least part of the length of a test pathway; preferably wherein the elongate cursor is slidable with respect to a test pathway.

11. A kit for quantitative measurement of a substance of interest in a liquid comprising a microfluidic liquid flow device according to any one of the preceding claims, in combination with a chart correlating extent of liquid travel in a test pathway with amount of substance of interest present in the liquid; and optionally buffers and/or reagents.

12. A method for performing a timed assay, comprising
i) providing a microfluidic liquid flow device according to any one of claims 1 to 11;
ii) causing or permitting liquid to flow in the test pathway and the timing pathway;
iii) measuring the extent of liquid flow in the test pathway in the predetermined time period set by the time taken for liquid in the timing pathway to reach the point of convergence with the test pathway.

13. A method according to claim 12, further comprising:
i) providing a liquid flow device according to claim 4;
ii) applying a known volume of sample to the test pathway through the sample port;
iii) applying a reagent to the device via the common inlet;
iii) determining the extent of liquid flow in the test pathway after reagent in the timing pathway reaches the point of convergence and stops flow in the test pathway.

14. A method according to claim 13 wherein the reagent is a chase buffer.

15. A method according to claim 12, further comprising:
i) providing a device according to any one of claims 5 to 8;
ii) applying a known volume of sample to the test pathway through the sample port;
iii) applying a reagent to the device via the common inlet;
iv) iii) determining the extent of liquid flow in the test pathway after reagent in the timing pathway reaches the point of convergence and stops flow in the test pathway;
v) comparing the distance travelled in the test pathway and reference pathway to compensate for variation in sample viscosity.

16. Use of a device according to any one of claims 1 to 10 in a method of regulating fluid flow, controlling timing of an assay, and compensating for variables
